# EUROPEAN PATENT APPLICATION

(11) **EP 1 679 515 A1**
(43) Date of publication of application: **12.07.2006**
(21) Application number: 04793079.7
(22) Date of filing: 21.10.2004
(51) Int. Cl.: G01N 33/53, G01N 37/00

(54) **BIO-ASSAY SUBSTRATE HAVING A POWER SUPPLY WIRE CONFIGURATION**

(30) Priority: 27.10.2003 JP 2003365745
(71) Applicant: SONY CORPORATION, Tokyo 141-0001 (JP)
(72) Inventor: YOSHIO, Akira, c/o Sony Corporation, Tokyo 141 (JP)
(74) Representative: Horner, David Richard
(86) International application number: PCT/JP2004/015967
(87) International publication number: WO 2005/040797

(57) **Abstract**

Provided is a configuration of feeder wirings for electrodes provided for reaction regions arranged in multiplicity on a disk-shaped substrate. Feeder wirings extended from a current passing portion (U) provided at a central portion of a substrate (A) to electrodes provided in reaction regions (R) arranged in multiplicity on the substrate so as to be fields for interactions between substances are each composed of a first wiring (1) led out from the current passing portion (U) toward the outer circumference of the substrate, a second wiring (2) branched from the first wiring, and a third wiring (3) branched further from the second wiring, and a voltage is impressed on the electrodes from the current passing portion (U) through the feeding wirings (1, 2, 3), to develop an electric field in the reaction regions (R).

## Description

### Technical Field

The present invention relates to a bioassay substrate for DNA chips or the like which is composed of a disk-shaped substrate. Specifically, the invention relates to the configuration of feeder wirings extended from a feeder portion provided at a predetermined position of a disk-shaped substrate to electrodes in respective reaction regions arranged on the substrate, particularly to the technology pertaining to the configuration of the feeder wirings in the vicinity of the reaction regions.

### Background Art

A first conventional technology pertaining to the present invention is the technology concerning a bioassay integrated substrate called a DNA chip or DNA microarray (hereinafter generically referred to as "DNA chip") in which predetermined DNAs are microscopically arranged by the microarray technique. The DNA chip technology is configured so that it is possible to comprehensively analyze inter-molecular reactions such as hybridization because a variety of and multiplicity of DNA oligo chains or cDNAs (complementary DNAs) are integrated on a glass substrate or silicon substrate (see Patent Document 1 (JP-A-Hei 4-505763), Patent Document 2 (JP-A-Hei 10-503841) and the like). Therefore, the DNA chips have been utilized for gene mutation analysis, SNPs (Single Nucleotide Polymorphisms) analysis, gene expression frequency analysis, etc., and have begun to be widely used in novel medicine development, clinical diagnosis, pharmaceutical genomics, legal medicine and other fields. In addition to the DNA chips, there have also been developed protein chips having proteins fixed on substrates, biosensors for analyzing various inter-substance interactions, and the like.

A second technology pertaining to the present invention is the technology concerning the actions of an electric field on substances present in an electrically charged state in a liquid phase. Specifically, a nucleotide chain (nucleic acid molecule) is known to extend or migrate under the action of an electric field in a liquid phase. The principle of this phenomenon is considered as follows; it is considered that the phosphate ion (negative charge) constituting the skeleton of the nucleotide chain and the hydrogen atom (positive charge) formed through ionization of water in the vicinity of the phosphate ion form an ion clouding, and polarization vectors (dipoles) generated by these negative and positive charges are as a whole set in one direction when a high-frequency high voltage is impressed thereon, resulting in elongation of the nucleotide chain; in addition, where an uneven electric field with electric lines of force concentrated on an area is impressed, the nucleotide chain migrates toward the location where the electric lines of force are concentrated (see Non-patent Document 1 (Seiichi Suzuki, Takeshi Yamanashi, Shin-ichi Tazawa, Osamu Kurosawa and Masao Washizu: "Quantitative analysis on electrostatic orientation of DNA in stationary AC electric field using fluorescence anisotropy", IEEE Transaction on Industrial Applications, Vol. 34, No. 1, pp.75 to 83(1998)). Besides, when a DNA solution is placed between micro-electrodes having a gap of several tens to several hundreds of micrometers and a high-frequency electric field of about 1 MV/m and 1 MHz is impressed thereon, dielectric polarization occurs in the DNA being present in a random coil form, resulting in that the DNA molecule is stretched rectilinearly in parallel to the electric field. It is known that, under this electrodynamic effect called dielectric migration, the polarized DNA is spontaneously attracted to the electrode end, to be fixed with its one end in contact with the electrode edge (see Non-patent Document 2 (Masao Washizu, "MINAGARA OKONAU DNA HANDORINGU (DNA Handling under Monitoring)", KASHIKA JOHO, Vol. 20, No. 76 (January 2000)).

The DNA chip technology at present has been spreading as a technology in which a multiplicity of reaction regions for providing the sites of interactions between substances in a liquid phase are preliminarily set on a substrate, and detecting nucleotide chains such as DNA probes are preliminarily fixed in the reaction regions so as to comprehensively analyze the hybridizations which are interactions between the detecting nucleotide chains and complementary target nucleotide chains.

In the case of carrying out the DNA chip technology, it is considered that if the detecting nucleotide chains (for example, DNA probes) can be fixed not in the rounded random coil form but in an extended state in the reaction regions, the bad influences of the so-called steric hindrance and the interferences (for example, adhesion and contact) between the detecting nucleotide chains and the surrounding surfaces, which might arise from the higher-order structures of substances, are excluded and, therefore, the efficiency of the hybridizations is enhanced.

Based on this novel idea, the present inventors have novelly devised a configuration in which an electrode functioning as a detecting surface is preliminarily disposed, and an electric field is impressed on a liquid phase in a reaction region between the electrode and an electrode opposed thereto. Then, the present inventors have successfully established a technology in which by this configuration it is possible to extend the detecting nucleotide chain present in the random coil form in the liquid phase by the action of the high-frequency electric field, to fix a terminal portion of the detecting nucleotide chain to the electrode edge, and to permit the hybridizations to progress efficiently.

However, in practicing this technology, a means to pass electric currents to the multiplicity of electrodes arranged on the substrate is indispensable. Therefore, in the case of adopting a disk-shaped substrate which is an advantageous substrate form for arranging a multiplicity of reaction regions having a electrode structure in various arrangement forms, there arise a problem that a multiplicity of wirings for feeding electric currents to the electrodes in all the reaction regions must be orderly laid on the substrate so that they will not interfere with each other.

In addition, in such a disk-shaped substrate, if standardized-shape reaction regions can be arranged along the circumferential direction, radially or spirally and they can be divided into blocks (grouped) in units each composed of a plurality of the reaction regions, the substrate space efficiency and the degree of integration of recorded information are enhanced, and it is possible to provide a bioassay substrate suitable for comprehensive and efficient analysis of genes and the like. Therefore there is a need to investigate such a wiring configuration suitable for arrangement of the reaction regions, particularly wiring configuration in the vicinity of the reaction regions.

Besides, at the time of reading the recorded information from the disk-shaped substrate on which the multiplicity of reaction regions are arranged, application of a rotation synchronizing servo or a tracking servo is considered to be carried out in the same manner as in an operation of reading recorded information from an optical disk such as a CD. In this case, it is necessary to ensure that exclusive-use signals or marks capable of being utilized for such servos can be read from the substrate, and the configuration on the substrate would thereby be made more complicated.

In view of the foregoing, an object of the present invention, for solving the novel problems generated attendant on the adoption of a disk-shaped substrate, is to provide a disk-shaped bioassay substrate in which a contrivance is applied to the configuration of feeder wirings, particularly a disk-shaped bioassay substrate in which a contrivance is applied to the configuration of feeder wirings in the vicinity of blocks each composed of a plurality of reaction regions.

### Disclosure of Invention

According to the present invention, first, there is provided a bioassay substrate being a disk-shaped substrate provided with reaction regions to be fields for interactions between substances and including electrodes provided in the reaction regions, a current passing portion provided at a central portion of the substrate, and feeder wirings for feeding electric currents from the current passing portion to the electrodes. In the bioassay substrate, the feeder wirings may be so configured that they can be grouped into (1) a "first wiring" led out toward the circumference of the substrate from the current passing portion, (2) a "second wiring" branched from the first wiring, and (3) a "third wiring" branched further from the second wiring, and a voltage may be impressed on the electrodes from the current passing portion through the feeder wirings so as to generate electric fields in the reaction regions.

In addition, according to the present invention, there is provided a configuration in which the third wiring in the bioassay substrate is extended from the second wiring toward the outside or inside in a radial direction. Besides, the third wirings may be utilized as terminal feeder wirings connectéd to the electrodes (in the reaction regions). Further, there is provided a configuration in which the third wirings are be extended on radial lines passing through the center of the substrate and are gradually increased in width from the substrate center side toward the substrate circumference side. This configuration ensures that the width of the reaction regions each arranged between the adjacent third wirings is constant.

There is provided a configuration in which the extensions of the center lines of the third wirings pass through the center of the substrate, and, in some cases, there is provided a configuration in which the third wirings themselves function also as the electrodes (in the reaction regions). In addition, a case where the electrodes formed in the reaction regions are composed of at least a pair of electrodes opposed to each other is assumed, and, in such a case, there can be provided a configuration in which the electrode on one side and the electrode on the other side of the opposed electrodes are connected respectively to different third wirings alternately branched from the second wiring.

The reaction regions arranged on the bioassay substrate according to the present invention can be grouped (divided into blocks) in units each composed of a predetermined number of the reaction regions, and all of the reaction regions on the substrate can freely be arranged efficiently in a pattern of concentric circles or a spiral line. This is because the bioassay substrate according to the present invention has a wiring configuration suitable for assuredly and efficiently feeding electric currents to all of the reaction regions constituting such an arrangement configuration.

Besides, when all of the reaction regions arranged on the substrate are arranged on radial lines passing through the center of the substrate, there is obtained a merit that a position detecting servo for the reaction regions can be easily carried out. Incidentally, when the second wirings are used as references for a rotation synchronizing signal or a tracking signal in reading the information recorded on the substrate, it is unnecessary to provide separate signal references on the substrate, which enables a simplification of the configuration or structure of the substrate.

### Brief Description of Drawings

Fig. 1 is a schematic diagram for illustrating a first embodiment (A) of the bioassay substrate according to the present invention.
Fig. 2 is an enlarged view showing a typical form of a reaction region (R).
Fig. 3 is a schematic diagram for illustrating a second embodiment (B) of the bioassay substrate according to the present invention.
Fig. 4 is an enlarged view of an essential part of a substrate region in the circle indicated by symbol Y in Fig. 3
Fig. 5 is a schematic diagram for illustrating a configuration in which a third wiring itself is used as an electrode.
Fig. 6 is a diagram showing a substrate (C) provided with a wiring configuration in which second wirings (2) led out from first wirings (1) are provided in a spiral pattern in overall perspective view.

### Best Mode for Carrying out the Invention

Now, preferred embodiments for carrying out the present invention will be described below referring to the accompanying drawings.

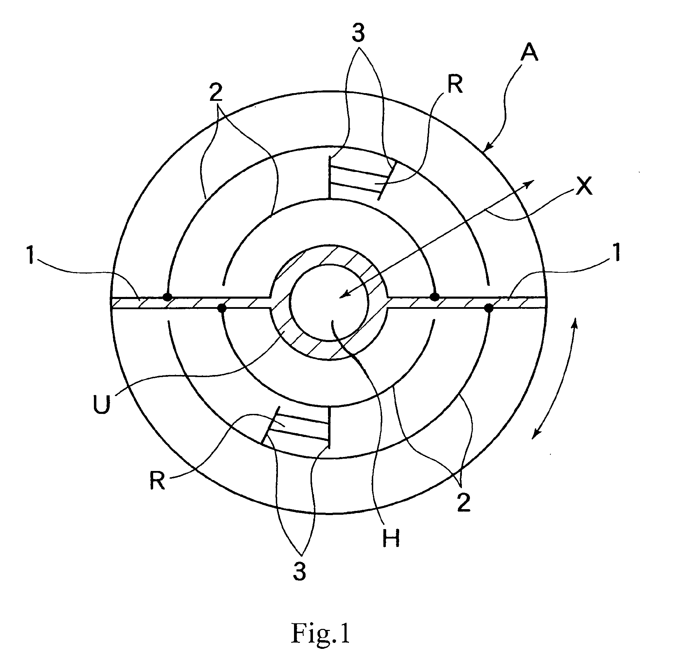

First, Fig. 1 is a schematic diagram for illustrating a first embodiment of the wiring configuration of a bioassay substrate according to the present invention. Incidentally, in all the drawings which will be used hereinafter, the configuration on a substrate will be shown in a simplified form, for convenience of illustration.

Symbol A in Fig. 1 denotes a bioassay substrate (hereinafter referred to simply as "the substrate") according to the present invention. The substrate A is formed of an insulating material such as a glass and a resin, and, as shown in Fig. 1, has a disk-like form in overall perspective view. On the substrate A, microscopic reaction regions R, R··· on the micrometer order in size are arranged in an exemplary pattern such as a radial pattern, a circumferential pattern, a spiral pattern, etc. and can be grouped.

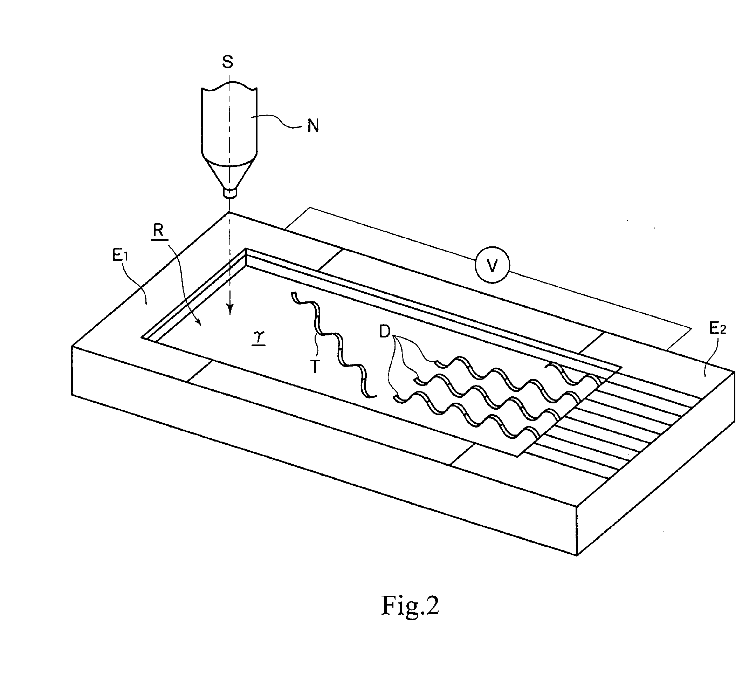

A typical form of these reaction regions R is enlargedly shown in Fig. 2. The reaction region R is a microscopic region providing a field for an interaction (for example, hybridization) between substances. In general, the reaction region R has a well shape (recess shape) in which a predetermined volume of a medium S such as a solution, a gel, etc. dropped from an ink jet nozzle N, a dispenser (not shown) or the like can be reserved or held.

In the reaction region R, for example, as shown in Fig. 2, a pair of opposed electrodes E1 and E2 are oppositely disposed on both sides of a reaction field r, or, though not shown, an electrode is singly disposed on a bottom surface or the like of the reaction field r. Incidentally, in the present invention, a reaction region provided with at least one electrode is dealt with, for convenience of description, and the specific arrangement position and shape of the electrode are not particularly limited.

Here, referring again to Fig. 1, the substrate A is provided in its central portion with a hole H having a predetermined aperture diameter. The hole H functions as a portion into which a chucking member (not shown) for holding or rotating the substrate A or a current passing jig is to be inserted. A ring-shaped current passing portion U is formed in the periphery of the hole H. The current passing portion U is configured to be capable of conduction with the current passing jig (not shown) to be inserted in the hole H.

From the current passing portion U, two first wirings 1, 1 functioning as main wirings of feeder wirings are extended along the radial direction X. Incidentally, the number of the first wirings 1 is not limited to two, and may be increased or decreased as required.

In the substrate A shown in Fig. 1, a multiplicity of second wirings 2, 2 ··· are extended from the first wirings 1, 1 so as to draw circular arcs in the circumferential direction. Furthermore, a multiplicity of third wirings 3 are led out and extended from the second wirings 2, alternately toward the inside and the outside in the radial direction (the direction denoted by X in Fig. 1). Incidentally, in Fig. 1, a total of four second wirings 2 and a total of four third wirings 3 are shown, simplifiedly for convenience of illustration.

The third wirings 3 play the role of terminal feeder wirings connected to the electrode E₁ and the electrode E₂ in each of the reaction regions R arranged in multiplicity on the substrate. Specifically, when the current passing jig (not shown) is inserted in the current passing portion U, a voltage is impressed on the electrode E₁ and the electrode E₂ in a predetermined reaction region R from the current passing portion U sequentially through the first wirings 1, the second wirings 2 and the third wirings 3.

The impressing of the voltage is conducted so as to ensure that detecting nucleotide chains D of DNA probes or the like and target nucleotide chains T having base sequences complementary to those of the detecting nucleotide chains D, which nucleotide chains are present in a free or fixed state in the reaction regions R, will be stretched or be moved under the action of dielectric migration (see Fig. 2). Incidentally, an electric field particularly suited to such a purpose is a high-frequency high-voltage AC electric field.

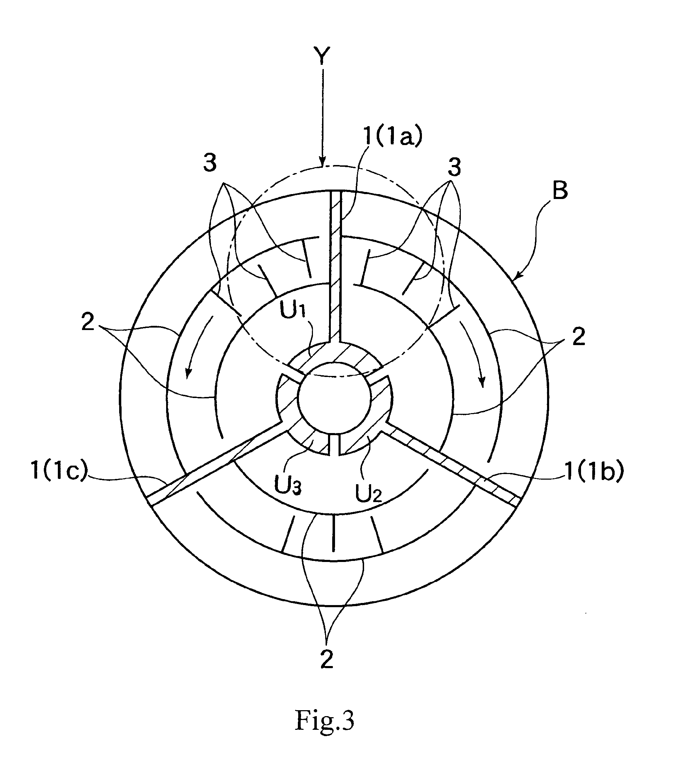

Fig. 3 is a schematic diagram for illustrating a second embodiment of the wiring configuration of the bioassay substrate according to the present invention.

In the substrate denoted by symbol B in Fig. 3, trisected current passing portions U₁, U₂ and U₃ are formed in the periphery of a hole H, and single first wirings 1a, 1b and 1c are extended in the radial direction X (see Fig. 1) respectively from the current passing portions U₁, U₂ and U₃. Incidentally, where the configuration with such divided current passing portions U₁, U₂ and U₃ is adopted, there is the merit that it is possible to select the current passing area on the substrate B.

In this substrate B, also, second wirings 2 are extended respectively from the first wirings 1a, 1b and 1c so as to draw circular arcs in the circumferential direction, and a multiplicity of third wirings 3 are extended from the second wirings 2, alternately toward the inside and the outside in the radial direction (the direction denoted by X in Fig. 1). Incidentally, in Fig. 3 also, the second wirings 2 and the third wirings 3 are shown in limited numbers, simplifiedly for convenience of illustration.

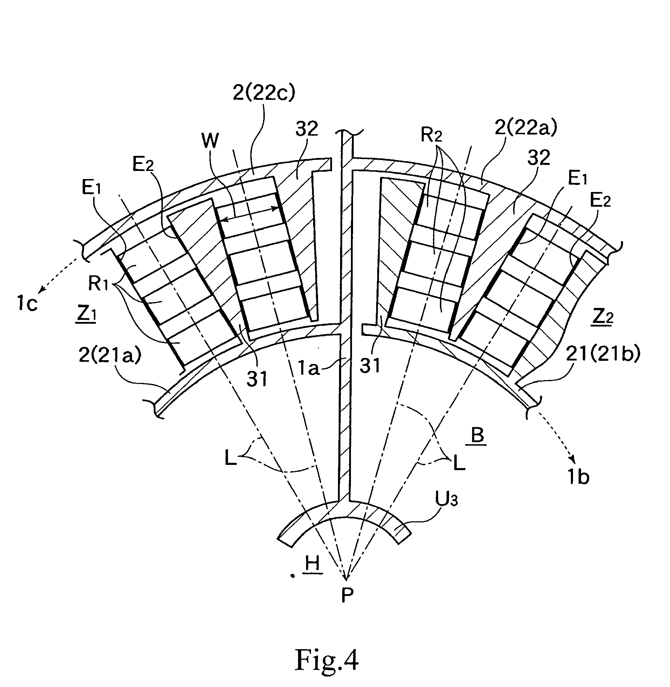

Next, Fig. 4 is an enlarged view of an essential part of a substrate region in the circle denoted by symbol Y in Fig. 3.

From the first wiring 1a led out and extended from the current passing portion U₃, a second wiring 21a is extended in the circumferential direction toward the adjacent first wiring 1c (see Fig. 3), and, on the outer circumference side thereof, a second wiring 22a is extended in the circumferential direction toward the adjacent first wiring 1b (see Fig. 3).

In addition, in Fig. 4, there are shown a second wiring 21b extended from the first wiring 1b (see Fig. 3) adjacent to the first wiring 1a to the vicinity of the first wiring 1a, and a second wiring 22c extended from the first wiring 1c (see Fig. 3) adjacent to the first wiring 1a to the vicinity of the first wiring 1a. As shown in Fig. 4, the second wiring 21b and the second wiring 21a are located on one circle, while the second wiring 22c and the second wiring 22a are located on another circle which is different in radius from but concentric with the one circle.

In a substrate region Z₁ defined between the inside second wiring 21a and the second wiring 22c directly on the outside thereof, a predetermined number of the reaction regions R₁ configured as shown in Fig. 2 are arranged. Similarly, in a substrate region Z₂ defined between the inside second wiring 21b and the second wiring 22a on the outside thereof, a predetermined number of the reaction regions R₂ are arranged.

The reaction regions R₁ and the reaction regions R₂ respectively constitute different reaction region groups (blocks) in which different DNA probes D (see Fig. 2) are fixed respectively. Incidentally, the method for grouping (division into blocks) the reaction regions is not limited to the one shown here.

Here, symbol 31 shown in Fig. 4 denotes a third wiring extended so as to be gradually increased in width toward the outside in the radial direction, and symbol 32 in the figure denotes a third wiring extended so as to be gradually decreased in width toward the inside in the radial direction.

The substrate region surrounded by the third wiring 31 formed in such a sector-like or roughly trapezoidal shape and the adjacent third wiring 32 is rectangular in shape, and, therefore, the reaction regions R (R₁, R₂) uniform in width W can be formed, and they can be arranged along radial lines L passing through the substrate center P. When such an arrangement configuration is adopted, the servo for determining the positions of all the reaction regions R is easy to carry out, which is preferable.

Though not shown, only either one of the adjacent third wirings 31 and 32 may be set in the above-mentioned shape having the stoutness (or width) gradually varied, whereby the reaction regions R (R₁, R₂) can be made uniform in width W. In such a configuration, however, the reaction regions R cannot be arranged along the radial lines L passing through the substrate center P.

As shown in Fig. 4, the electrodes E₁ and E₂ disposed in each reaction region R are connected with the third wirings 31 and 32, so that a voltage can be impressed on the reaction region R.

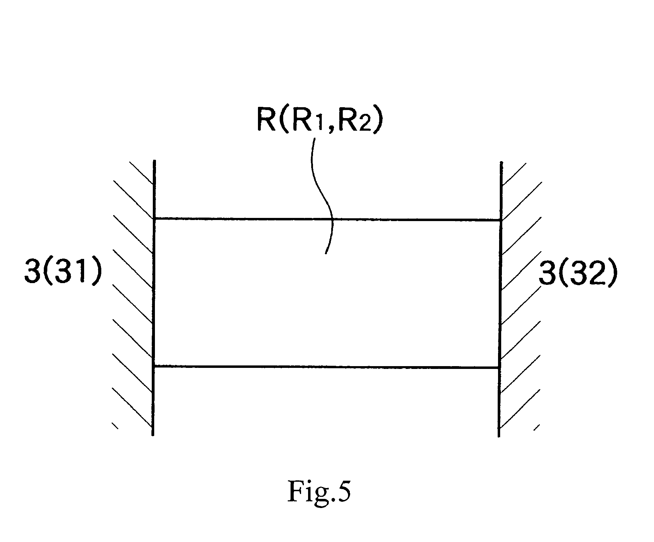

Incidentally, as shown in Fig. 5, the third wirings 3 (31, 32) themselves may be formed to front on the reaction region, whereby the third wirings 3 (31, 32) themselves can be utilized as electrodes. The wiring configurations shown in Figs. 4 and 5 can be applied to the above-mentioned substrate A and a substrate B.

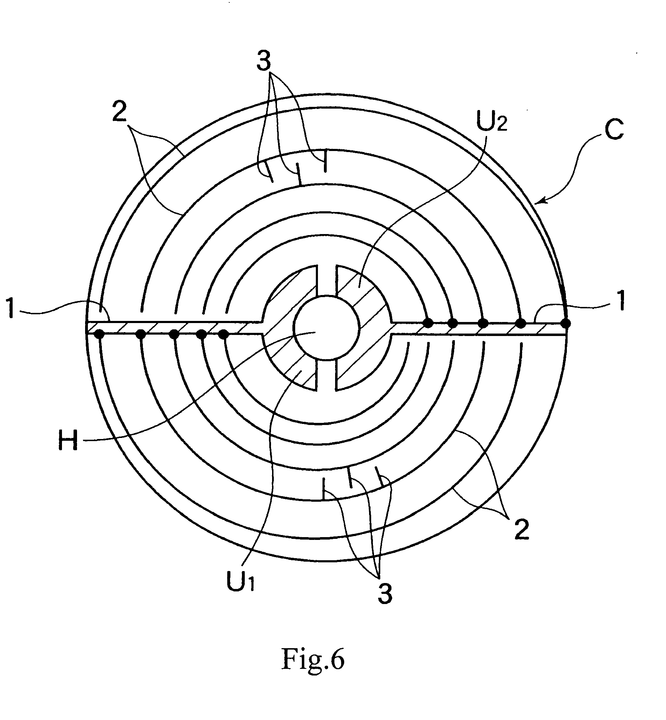

Next, Fig. 6 shows a substrate C according to a third embodiment having a wiring configuration in which second wirings 2 led out and extended from first wirings 1 are provided in a spiral pattern in overall perspective view.

In the substrate C, bisected current passing portions U₁ and U₂ are provided in the vicinity of a hole H, and single first wirings 1 are extended in the radial direction X (see Fig. 1) respectively from the current passing portions U₁ and U₂. From the second wirings 2 extended from the first wirings 1, 1 in the spiral pattern in overall perspective view, a multiplicity of third wirings 3 are extended alternately toward the inside and the outside in the radial direction (the direction denoted by X in Fig. 1).

The feeder wirings laid on the disk-shaped substrates A, B and C as above can be used also as references for a rotation synchronizing signal, or as references for a tracking signal, used at the time of reading the recorded information. As a result, the need to provide the substrate with exclusive-use signals or marks, such as a group of pits or a group of bar codes, for obtaining the rotation synchronizing signal and the tracking signal is eliminated, so that the substrate can be more simplified in configuration.

The feeder wirings as above-described may be composed of a single wiring layer or a plurality of wiring layers, according to the purpose or the substrate configuration. For example, a configuration may be adopted in which the wiring layer is divided into two layers, and the feeding to the one-side electrode in a reaction region R and the feeding to the other-side electrode in the reaction region R are achieved through the different wiring layers, respectively.

According to the bioassay substrate of the present invention, in the case where the reaction regions provided with electrodes are arranged on a disk-shaped substrate, particularly in the case where the reaction regions are arranged on the substrate in a grouped state and in a high density, the feeder wirings to be connected to the electrodes can be laid on the substrate in a high density and orderly. As a result, it is easy to form the reaction regions in a high density, irrespectively of the inner or outer position in the radial direction of the substrate. In addition, the efficient wiring configuration shortens the length of wiring, so that wiring resistance can be reduced.

Besides, according to the wiring configuration adopted in the bioassay substrate of the present invention, the feeding to the electrodes provided for the reaction regions on the substrate can be assuredly achieved, irrespectively of the arrangement configuration and form of the electrodes, so that the degree of freedom in substrate design can be enhanced.

It is possible to arrange the reaction regions provided with the electrodes and having the same shape and size in the whole area on a substrate, and to feed the reaction regions with electricity.

The feeder wirings laid on the disk-shaped substrate can be used also as references for a rotation synchronizing signal, or references for a tracking signal, used at the time of reading the recorded information. As a result, the need to provide the substrate with exclusive-use signals or marks, such as a group of pits or a group of bar codes, for obtaining the rotation synchronizing signal and the tracking signal is eliminated, so that the substrate can be more simplified in configuration.

### Industrial Applicability

The present invention can be utilized as a DNA chip or other bioassay substrate, particularly a disk-shaped bioassay substrate, on which reaction regions provided with electrodes are arranged in multiplicity.

## Claims

1. A bioassay substrate being a disk-shaped substrate provided with reaction regions to be fields for interactions between substances and comprising electrodes provided in said reaction regions, a current passing portion provided at a central portion of said substrate, and feeder wirings for feeding electric currents from said current passing portion to said electrodes, wherein:
said feeder wiring is included a first wiring led out toward the circumference of said substrate from said current passing portion, a second wiring branched from said first wiring, and a third wiring branched further from said second wiring; and
a voltage is impressed on said electrodes from said current passing portion through said feeder wirings so as to generate electric fields in said reaction regions.

2. The bioassay substrate as set forth in claim 1, wherein said third wiring is extended from said second wiring toward the outside or inside in a radial direction.

3. The bioassay substrate as set forth in claim 2, wherein said third wirings are terminal feeder wirings connected to said electrodes.

4. The bioassay substrate as set forth in claim 3, wherein said third wirings are extended on radial lines passing through the center of said substrate and are gradually increased in width from the substrate center side toward the substrate circumference side so that the width of said reaction regions each arranged between the adjacent third wirings is constant.

5. The bioassay substrate as set forth in claim 1, wherein the extensions of the center lines of said third wirings pass through the center of said substrate.

6. The bioassay substrate as set forth in claim 1, wherein said third wirings function also as said electrodes.

7. The bioassay substrate as set forth in claim 1, wherein said electrodes are comprised of at least a pair of electrodes opposed to each other, and the electrode on one side and the electrode on the other side of said opposed electrodes are connected respectively to different third wirings alternately branched from said second wiring.

8. The bioassay substrate as set forth in claim 1, wherein said reaction regions are grouped in units each composed of a predetermined number of said reaction regions.

9. The bioassay substrate as set forth in claim 1, wherein all of said reaction regions on said substrate are arranged in a pattern of concentric circles or a spiral line.

10. The bioassay substrate as set forth in claim 1, wherein all of said reaction regions are arranged on radial lines passing through the center of said substrate.
